# DEMANDE DE BREVET EUROPEEN

(11) **EP 1 705 192 A1**
(43) Date de publication de la demande: **27.09.2006**
(21) Numéro de dépôt: 06290462.8
(22) Date de dépôt: 22.03.2006
(51) Int. Cl.: C08B 37/08, C07H 3/00, A61K 31/726

(54) **Nouvelles fractions issues de chondroïtines et leur utilisation en tant que médicament**

(30) Priorité: 22.03.2005 FR 0502810
(71) Demandeur: Labotaroire Genevrier, 06901 Sophia Antipolis Cedex (FR)
(72) Inventeur: Vacher, Dominique, Parc de Sophia-Antipolis 06600 Anitbes (FR)
(74) Mandataire: Burtin, Jean-François

(57) **Abrégé**

L'invention se rapporte au domaine des nécessités de la vie et plus particulièrement au domaine de la chimie biologique.

Elle a spécifiquement pour objet la production et la caractérisation notamment après couplage avec un réactif chromophore fluorescent, des blocs d'acides chondroïtine sulfuriques extraits de cartilages d'origine porcine, bovine ou ichtyque.

La présente invention repose également sur les propriétés biologiques possédées par les blocs de composés dérivés d'acides chondroïtine sulfuriques, extraits de cartilage.

Utilisation en thérapeutique de ces blocs d'acides chondroïtine sulfuriques notamment comme médicament des cartilages et pour lutter contre les troubles articulaires occasionnés par les processus de vieillissement.

## Description

La présente invention se rapporte au domaine des nécessités de la vie et plus particulièrement au domaine de la chimie biologique.

La présente invention a plus particulièrement pour objet de nouvelles fractions issues d'acides chondroïtine sulfuriques, extraits de cartilages d'origine bovine, porcine et ichtyque.

Elle a spécifiquement pour objet des blocs d'acides chondroïtine sulfuriques sulfatés en 4 et en 6 isolés par des méthodes chimiques qui permettent la production et la caractérisation des dits blocs d'acides chondroïtine sulfuriques sulfatés en 4 et en 6 provenant des différents types de cartilage.

Des études antérieures ont défini la composition des chondroïtines, notamment au nom du Demandeur dans la demande de brevet. C'est ainsi qu'on a déterminé que les chondroïtines isolées sont constituées essentiellement de disaccharides sulfatés en position 2, 4, 6 et/ou de disaccharides di-sulfatés en position 2, 6. Elles peuvent inclure des disaccharides non sulfatés. Ce sont des facteurs de conservation et de régénération des cartilages. Ces chondroïtines sont obtenues par clivage enzymatique des protéoglycanes (PG) déjà sulfatées, contenus dans les cartilages de différentes espèces animales, et notamment d'origine porcine, bovine ou ichtyque.
On a pu ainsi attribuer aux chondroïtines la structure linéaire suivante :

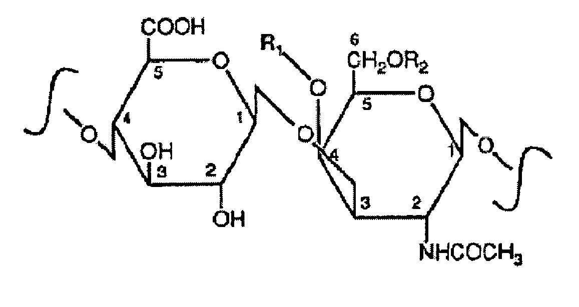

| | R₁ | R₂ |
|---|---|---|
| α-D-GlcA*p*-(1->3)-β-D-Gal*p*-NAc 4S Chondroitin 4-sulfate | SO₃H | H |
| α-D-GlcA*p*-(1->3)-β-D-Gal*p*-NAc 6S Chondroitin 6-sulfate | H | SO₃H |

Selon N.Volpi Current Drug Target (2004) 4, 119-127, les chondroïtine sulfates constituent des polyanions et certaines de leurs propriétés sont liées à leur forte charge qui est capable d'attirer l'eau dans les tissus et de contribuer à leur hydratation. Les chaînes d'acides chondroïtine sulfuriques sont synthétisées par des cellules. Elles sont liées d'une manière covalente aux protéines qui sont sécrétées dans la matrice extracellulaire, sous forme de protéoglycanes. Plusieurs familles de protéoglycanes, incluant des chaînes d'acides chondroïtine sulfuriques, ont été caractérisées, dont deux des familles les plus importantes de protéoglycanes trouvées dans la matrice extracellulaire des tissus connectifs : la famille des aggrécans et la famille des protéoglycanes riches en motifs répétitifs à base de leucine.

Les chondroïtine sulfates sont formées d'une répétition d'acide glucuronique (GlcA) et de N-acétyl-galactosamine (GalNac) [-4)GlcA(β1-3)GalNAc(β1-]ₙ.
Elles peuvent être sulfatées en position 4 ou/et 6 de GalNAc et en position 2 de GlcA. Les résidus de GalNAc sulfatés en position 4 ou 6 sont plus fréquemment rencontrés. Des résidus non sulfatés peuvent également être trouvés. Les résidus de GalNAc sulfatés en position 4,6 ainsi que les résidus d'acide uronique sulfatés en position 3 sont des structures beaucoup plus rares.

On connaissait déjà des mélanges d'acides chondroïtine sulfuriques et de glycosaminoglycanes sulfatés commercialisés sous le nom d'Arteparon® et de Structum®. Ces mélanges contiennent des chondroïtines plus ou moins identifiées et principalement de l'acide chondroïtine 6-sulfurique. Toutefois aucune méthode d'analyse n'avait pu déterminer avec certitude la structure de telles chondroïtines.
L'Arteparon ® est un dérivé de glycosaminoglycane sulfaté comprenant de la galactosamine, de la glucosamine, de l'acide glucuronique et des sulfates dans les proportions (calculés en µmoles/100 mg) : 93.4, 11.1, 94.6, et 425.0, respectivement. (cf Biochemical Pharmacology vol.29.pp.1723-1727.© Pergamon Press Ltd.1980.Printed in Great Britain "Inhibition of Human elastase from polymorphonuclear leucocytes by a glycosaminoglycan polysulfate (Arteparon)" Antonio Baici, Prathima Salgam, Kurt Fehr and Albert Böni, Department of Rheumatology, University Hospital, Gloriastrasse 25, CH-8091 Zurich, Switzerland).
Les composants sulfates contiennent environ quatre groupes -OSO₃⁻ par unité de disaccharide. Par les compositions, on a pu calculer une moyenne de poids par unité de disaccharide, de 700 environ.
Les fractions connues d'Arteparon ® sont GAGPS A 77038, GAGPS A 79067, GAGPS L 980 B, GAGPS L 980, GAGPS L980 A.
Les poids molaires de ces fractions d'Arteparon ® connus sont de 2100, 3100, 6500, 9900 et 17000. Ils contiennent un nombre moyen d'unités monomères respectivement de 6, 9, 19, 28, 48 et une longueur de chaînes en (A°) de 30, 45, 95, 140 et 240.
Le Structum ® est un mélange polydisperse de chondroïtine-4-sulfate et de chondroïtine-6-sulfate extraits de cartilage (cf Chem-Biol.Interactions, 51 (1984)1-11 Elsevier scientific Publishers Ireland Ltd « Interaction between human leukocyte elastase and chondroitin sulfate » Antonio Baici and Paola Bradamante, Department of Rheumatology, University Hospital, Gloriastrasse 25, CH-8091, Zurich (switzerland)).
Ces fractions connues d'Arteparon ® et de Structum ® sont plus ou moins identifiés mais le schéma de sulfatation sous la forme de blocs d'acides chondroïtine sulfuriques était jusque là inconnu. C'est sur cette constatation que repose la présente invention et plus précisément sur l'identification de blocs d'acide chondroïtine sulfuriques sulfatés en 4 et en 6 .

On connaissait déjà des méthodes de caractérisation des acides chondroïtine sulfuriques telles que la RMN (cf Eur.J.Biochem.268,1181-1189 (2001) « Characterization of oligosaccharides from the chondroitin sulfates 1H-NMR and 13C-NMR studies of reduced disaccharides and tetrasaccharides » ThomasN.Huckerby, Robert M.Lauder, Gavin M.Brown, Ian A.Nieduszynski, Karen Anderson, Joanne Boocock, Patricia L.Sandall and Stephane D.Weeks). Les chondroïtine sulfates fragmentées par les enzymes chondroïtin sulfate ABC endolyase et chondroïtin ACII lyase étaient isolées après réduction en 2-déoxy-2N-acétylamino-D-galactitol. La RMN [¹H] et RMN [¹³C] ont pu aider à la caractérisation des composants structuraux des polymères de chondroïtine sulfates et des dermatans sulfates, mais cette méthode n'a pas permis d'identifier les différents blocs d'acides chondroïtine sulfuriques de manière satisfaisante.

Le Demandeur a trouvé maintenant que le schéma de sulfatation des chondroïtines sulfates était toujours répétitif et n'était pas le fait d'un hasard. Ainsi les acides uroniques sulfatés en position 2 se trouvent seulement entre un GalNac sulfaté en 4 sur le coté réducteur et un GalNAc sulfaté en 6 sur le coté non réducteur. On a constaté également que les acides chondroïtine sulfuriques sulfatés en 4 isolés et identifiés sont organisés en blocs d'acides chondroïtine sulfuriques et plus précisément des blocs de 2 disaccharides, de 3 disaccharides, de 4 disaccharides, de 5 disaccharides et notamment des enchaînements 6-4, 6-4-4, 6-4-4-4, 6-4-4-4-4 . Entre ces blocs de disaccharides sulfatés en 4, il existe également des disaccharides non sulfatés et des disaccharides sulfatés en 6.
Selon la présente invention, on peut inclure en outre des blocs de 6 disaccharides, des blocs de 7 disaccharides, des blocs de 8 disaccharides, des blocs de 9 disaccharides ou même des blocs de 10 disaccharides.
Pour cette détermination, on utilise des échantillons de chondroïtine d'origine bovine et des échantillons de chondroïtine d'origine porcine. Les analyses ont été répétées 3 fois. La détermination de la structure de ces blocs et l'identification des acides chondroïtine sulfuriques a été réalisée par digestion enzymatique en utilisant la chondroïtin C-lyase et détermination par chromatographie d'échange d'anions sur résine et détection par des méthodes physiques ou chimiques comme la fluorescence après couplage avec l'acide 2-aminobenzoïque. La chondroïtin C-lyase est un enzyme qui ne coupe qu'en amont des liaisons des disaccharides sulfatés en 6 ce qui explique les différents blocs obtenus.

Les méthodes d'analyse utilisées sont présentés dans la partie expérimentale.
L'analyse présentée à l'exemple 2 a été menée sur des lots différents pour une même espèce. L'analyse porte sur la comparaison des temps d'élution et sur la surface des pics en HPLC.

Ainsi on a constaté que pour tous les lots de blocs d'acides chondroïtine sulfuriques d'origine bovine, les temps d'élution ainsi que la surface des pics sont pratiquement identiques d'un lot à l'autre.
On a également comparé les différents blocs d'acides chondroïtine sulfuriques entre les différentes espèces. Ces analyses sont présentées dans l'exemple 3.
Ainsi, 80% des pics dans l'étude sur les blocs d'acides chondroïtine sulfuriques d'origine bovine et 75% dans l'étude de blocs d'acides chondroïtine sulfuriques d'origine porcine présentent des enchaînements définis et identifiés.

L'invention concerne également un procédé de production des blocs d'acides chondroïtine sulfates qui consiste à soumettre les acides chondroïtine sulfates extraits de cartilage à l'action d'un enzyme du type chondroïtineC-lyase et à soumettre les liqueurs d'hydrolyse à une chromatographie sur une colonne échangeuse d'anion puis élution par des liqueurs alcalines comportant un gradient de chlorure de sodium puis lavage de la colonne et contrôle de l'élution des oligosaccharides par fluorescence. Le gradient de chlorure de sodium varie de 0 à 30% de chlorure de sodium 2M.
Le chromatogramme est donné en annexe et est expliqué en exemple 4. Les blocs d'acide chondroïtine sulfuriques sont clairement définis par le Demandeur.

L'invention a encore pour objet l'utilisation de ces blocs d'acides chondroïtine sulfuriques identifiés et séparés par le demandeur pour des applications thérapeutiques et notamment pour l'inhibition de l'élastase, par exemple pour l'inhibition de l'élastase des leucocytes humains.

On a démontré précédemment que les acides chondroïtine mono- ou disulfuriques manifestaient "in vitro" sur des chondrocytes humains des augmentations dans le taux de synthèse des aggrécans. On connaissait déjà l'activité anti-élastase des acides chondroïtine sulfuriques bruts. La présente demande a pour objet l'utilisation de l'activité biologique des blocs d'acides chondroïtine sulfuriques, à des degrés supérieurs.

L'élastine est un composant de la matrice extracellulaire ( charpente qui entoure les cellules). Elle est la protéine principale des fibres élastiques des artères, des veines, du poumon, de la peau...On sait que les fibres élastiques s'altèrent au cours du vieillissement et des maladies acquises que sont l'artériosclérose (vieillissement des artères), l'athérosclérose, les anévrismes ...

L'élastase contenue dans les granulations azurophiles des leucocytes neutrophiles est une protéinase qui, libérée dans une articulation, a un pouvoir de dégradation des tissus connectifs (arthrite rhumatoïde). C'est un enzyme capable de dégrader les quatre composants de la matrice extracellulaire : collagène, élastine, protéoglycanes et glycoprotéine structurale.
Des études in vitro sur le catabolisme du cartilage ont montré que l'élastase dégrade dans une première phase les protéoglycanes et dans une seconde phase le collagène.

L'activité de l'élastase libre n'était pas détectée dans le liquide synovial des patients souffrant d' arthrite rhumatoïde mais il a été possible de démontrer que l'enzyme était présent, dans la couche superficielle dans les articulations du cartilage humain.

L'élastase des leucocytes peut dégrader les 4 sous-classes des IgG et IgM et joue un rôle dans l'immunopathologie des maladies inflammatoires.

L'élastase est un enzyme qui dégrade les protéines à serine. Son action est neutralisée par l'alpha1-antitrypsine, une anti-protéase du groupe des serpines.

Les granulations des polymorphonucléaires neutrophiles contiennent une large quantité d'élastase. Cet enzyme peut dégrader presque tous les composants de la matrice extracellulaire et même attaquer les cellules intactes.
Les élastases neutrophiles actives peuvent être détectées dans les liquides de surfaces d'inflammation des arthrites rhumatoïdes. De plus, il a été montré que les enzymes sont capables de pénétrer dans le cartilage et ainsi de les dégrader.

La LEI (leucocyte elastase inhibitors) est une protéine appartenant à la famille des serpines, qui regroupe des inhibiteurs de protéases d'un type particulier impliqués dans de nombreux processus physiologiques et pathologiques, notamment de dégradation.

Dans la littérature, on a décrit comment vérifier l'activité inhibitrice des acides chondroïtine sulfuriques des sélaciens (requin) sur l'élastase, en utilisant comme substrat le N -tert-butyloxycarbonyl p-nitrophényle. L'élastase hydrolyse le N -tert-butyloxycarbonyl p-nitrophényle mais en présence des acides chondroïtine sulfuriques des sélaciens, le N -tert-butyloxycarbonyl p-nitrophényle reste intact.

L'élastase lysosomale humaine de leucocytes polymorphonucléaires est inhibée par les polysulfates GAG comme l' Arteparon ®.
En effet, une faible quantité d'Arteparon ® allant de 0.1 à 1 µg/ml de poids moléculaire 10000, peut inhiber l'élastase lysosomale humaine.
L'Arteparon est un dérivé de glycoseaminoglycane sulfaté contenant de la galactosamine, de la glucosamine, de l'acide glucuronique et des sulfates.

Cette inhibition se fait par des forces électrostatiques. En effet, les élastases de nature cationique s'attachent aux GAGs qui sont anioniques. Cependant les complexes élastase-Arteparon ne précipitent pas et sont dissociables réversiblement par une haute concentration de sels.

D'autres agents d'inhibition des élastases existent, comme ceux connus commercialement sous le nom de Structum®.
Le Demandeur a constaté que le pourcentage d'inhibition de l'activité des élastases des leucocytes humains augmente en fonction de la densité de charge des chondroïtine sulfates. En particulier, l'inhibition de l'activité enzymatique diminue avec le pourcentage de disaccharides non sulfatés et augmente avec la proportion de disaccharides 2.6 sulfatés.
Pour une même densité de charge, le pourcentage d'inhibition de l'activité des élastases des leucocytes humains augmente avec des acides chondroïtine sulfuriques de forte masse.

Le Demandeur est parvenu à isoler et à identifier les différents blocs d'acides chondroïtine sulfuriques de sulfatation en 4 et en 6 des cartilages et ainsi a pu les utiliser dans des compositions pharmaceutiques qui, par exemple, ont un effet anti-élastase très important.

Les résultats expérimentaux permettent de supposer que l'affinité des différents motifs des chondroïtine sulfates dépend de l'enchaînement de ces blocs, du nombre de molécules de chondroïtine sulfates et de la conformation de la molécule. Il en résulte des niveaux d'activité clairement différents.

Les essais ont également montré qu'après digestion enzymatique et chromatographique par échange d'anions on constate une relative similarité entre les chondroïtines d'origine aviaire et d'origine porcine, alors que les chondroïtines d'origine bovine présentent après digestion une proportion de molécules sulfatées et une répartition et répartition des masses moléculaires nettement différente.

Les blocs d'acides chondroïtine sulfuriques identifiés selon la présente invention sont utilisés à des fins thérapeutiques, notamment par voie orale ou parentérale, sous forme de compositions pharmaceutiques telles que comprimés, dragées, gélules, pilules, cachets, poudres aromatisées ou non, ou d'injections destinées à la voie intramusculaire, sous forme d'ampoules, de flacons monodoses ou de seringue auto-injectables.

Les blocs d'acide chondroïtine sulfurique selon l'invention constituent un médicament précieux pour lutter contre les maladies articulaires, notamment celles liées au vieillissement.

Les doses utilisées peuvent varier considérablement en fonction de l'ancienneté ou de l'évolution de la maladie. Les blocs d'acides chondroïtine sulfuriques isolés et identifiés selon l'invention sont pratiquement dépourvus de toxicité et trouvent de ce fait un emploi très large en thérapeutique.

Une dose usuelle pour la voie orale ou parentérale s'échelonne de 0.1 g à 2 g par prise unitaire et de préférence de 0.2 g à 0.5 g par prise.

### EXEMPLES

### Exemple 1 : matériels et méthodes utilises

### a) Matériel et méthodes utilisés :

Les produits utilisés sont :
- Acide acétique glacial
- DMSO
- Acide 2-amino benzoïque
- Chondroïtin C lyase
- Hydroxyde de sodium 46/48%
- Produits chimiques de qualité pour l'analyse

### b) Méthode utilisée pour dépolymériser les chaînes de GAG (glycosaminoglycane):

Les chondroïtine sulfates sont mis en suspension à 50 mg/ml dans une solution d'acétate d'ammonium de concentration 0.1M, à pH 8 et dépolymérisés par 5U/mg de chondroïtin C-lyase pendant 7 jours à 22 °C avant que l'enzyme soit désactivé par chauffage à 100°C pendant 1 minute.

### c) Marquage par fluorescence :

Les chaînes CS (chondroïtine sulfate) non réduites en plus des oligosaccharides sont marquées en fluorescence avec l'acide 2-amino benzoïque.
2 µl du mélange marqué est additionné à l'aliquot dépourvu de sel et lyophilisé, mais réduit. Les oligosaccharides et le mélange sont chauffés à 65 °C pendant 2 heures. Puis l'échantillon marqué est remis en suspension dans 100 µl de d'eau et analysé par HPAEC (high pH anion exchange chomatography).
Le mélange marqué est préparé par les réactifs précédemment décrits
- ajout de 150 µl d'acide acétique glacial à 350 µl de DMSO
- ajout de 120 µl de ce mélange à 8.8 mg d'acide 2-amino benzoïque
- ajout de 100 µl de ce mélange à 11.8 mg de cyanoborohydrure de sodium
Le cyanoborohydrure est un agent réducteur qui réduit sélectivement une fonction acide en fonction alcool.

### d) HPAEC (high pH anion exchange chomatography) :

La composition des chaînes CD/DS (acide chondroïtine sulfurique/ acide dermatan sulfurique) a été examiné par HPAEC (high pH anion exchange chomatography).
Les oligosaccharides sont séparés en utilisant la colonne de résine commercialisée sous la dénomination Dionex As4-SC (4mm*250mm) maintenue à 50°C et s'écoulant à un débit de 2ml/min.
Une phase isocratique de 15% de NaOH 1M / 85%H2O / 0%NaCl 2M est suivie d'une phase de gradient d'élution de 0-30% NaCl 2M pendant 60 minutes avec 15 % NaOH .
La colonne est ensuite lavée avec 30% NaOH 1M / 10% H2O / 60% NaCl 2M .Puis une phase de rééquilibration dans les mêmes conditions de départ est suivie.
Les oligosaccharides élués sont révélés par fluorescence en utilisant l'équipement Jasco Fluorescent avec une excitation maximum à 315 nm et une émission maximum à 400 nm.

### Exemple 2 : résultats pour différents lots d'une même espèce :

**Tableau des moyennes des résultats des temps d'élution :**

| Porcine | | | Bovine | | |
|---|---|---|---|---|---|
| Pics | Moyennes | DS | Pics | Moyennes | DS |
| 6 | 14.28 | 0.43 | 6 | 13.67 | 0.69 |
| 6-4 | 24.97 | 0.51 | 6-4 | 24.35 | 0.76 |
| 6-4-4 | 33.44 | 0.64 | 6-4-4 | 32.66 | 0.88 |
| | | | 6-4-4- | | |
| 6-4-4-4 | 39.94 | 0.66 | 4 | 39.11 | 0.86 |
| 6-4-4- | | | 6-4-4- | | |
| 4-4 | 45.14 | 0.63 | 4-4 | 44.18 | 0.88 |

**Tableau des résultats des temps d'élution pour les différents lots d'origine porcine :**

| P1G | | |
|---|---|---|
| Pics | Moyennes | DS |
| 6 | 26.44 | 2.04 |
| 6-4 | 12.46 | 0.53 |
| 6-4-4 | 5.36 | 0.94 |
| 6-4-4-4 | 2.83 | 0.35 |
| 6-4-4-4-4 | 1.94 | 0.06 |

| P2B | | |
|---|---|---|
| Pics | Moyennes | DS |
| 6 | 23.01 | 1.95 |
| 6-4 | 13.67 | 1.82 |
| 6-4-4 | 6.53 | 1.09 |
| 6-4-4-4 | 3.35 | 0.36 |
| 6-4-4-4-4 | 1.76 | 0.18 |

| P3 | | |
|---|---|---|
| Pics | Moyennes | DS |
| 6 | 28.61 | 1.46 |
| 6-4 | 15.65 | 1.73 |
| 6-4-4 | 7.50 | 0.94 |
| 6-4-4-4 | 4.55 | 0.83 |
| 6-4-4-4-4 | 2.51 | 0.25 |

| P4 | | |
|---|---|---|
| Pics | Moyennes | DS |
| 6 | 23.77 | 2.70 |
| 6-4 | 15.02 | 2.29 |
| 6-4-4 | 7.33 | 1.15 |
| 6-4-4-4 | 3.55 | 0.63 |
| 6-4-4-4-4 | 2.27 | 0.30 |

| P5 | | |
|---|---|---|
| Pics | Moyennes | DS |
| 6 | 27.45 | 2.14 |
| 6-4 | 18.69 | 1.15 |
| 6-4-4 | 6.51 | 0.07 |
| 6-4-4-4 | 4.69 | 0.05 |
| 6-4-4-4-4 | 2.65 | 0.19 |

| P36 | | |
|---|---|---|
| Pics | Moyennes | DS |
| 6 | 26.89 | 2.64 |
| 6-4 | 15.84 | 1.34 |
| 6-4-4 | 6.34 | 0.39 |
| 6-4-4-4 | 3.97 | 0.75 |
| 6-4-4-4-4 | 2.73 | 0.25 |

**Tableau des moyennes des résultats des surfaces des pics :**

| Porcine | | | Bovine | | |
|---|---|---|---|---|---|
| Pics | Moyennes | DS | Pics | Moyennes | DS |
| 6 | 26.03 | 2.96 | 6 | 36.86 | 2.26 |
| 6-4 | 15.22 | 2.50 | 6-4 | 16.41 | 1.24 |
| 6-4-4 | 6.59 | 1.11 | 6-4-4 | 7.17 | 0.50 |
| | | | 6-4-4- | | |
| 6-4-4-4 | 3.83 | 0.86 | 4 | 4.66 | 0.45 |
| 6-4-4- | | | 6-4-4- | | |
| 4-4 | 2.31 | 0.42 | 4-4 | 2.38 | 0.28 |

Les lots présentent des surfaces de pics similaires pour les différents acides chondroïtine sulfuriques dans le cas de l'espèce bovine.
Par contre une variation, pour les pics correspondants au tétrasaccharide 6-4, existe entre les différents lots de l'espèce porcine.

### Exemple 3 : résultats et comparaisons des 2 espèces :

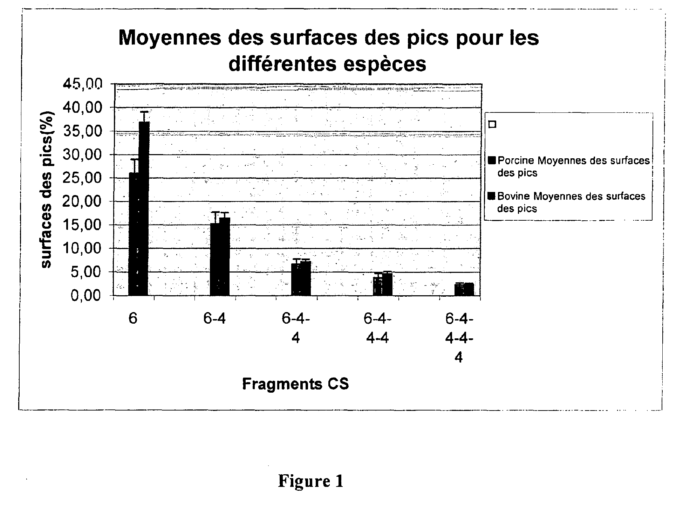

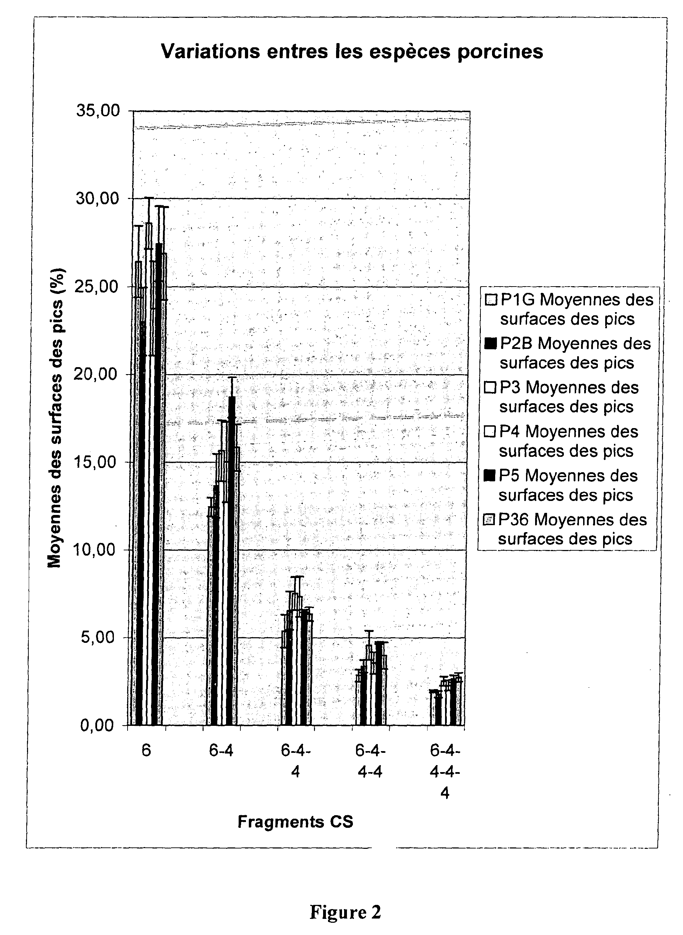

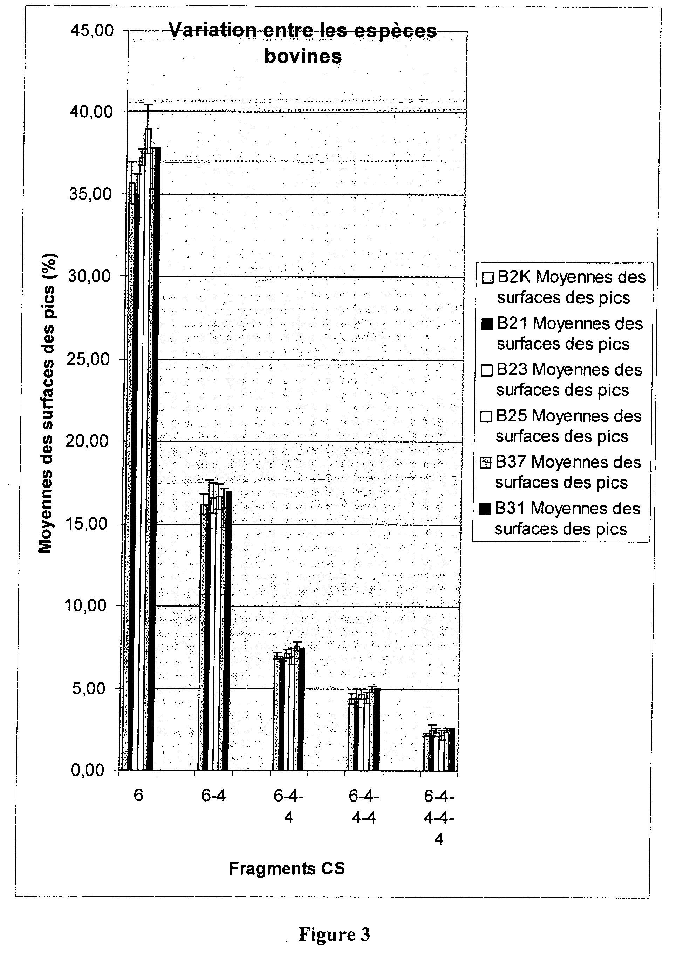

Les résultats de la figure 1 montrent une nette différence des surfaces des pics des blocs de disaccharides 6-sulfatés selon les espèces bovine et porcine. En effet, on note une abondance des blocs de disaccharides 6-sulfatés de l'ordre de 40% ±2.3% pour l'espèce bovine, de 25% ±3% pour l'espèce porcine.
La quantité de blocs de dérivés 6-sulfurique est dépendante comme pour les blocs de dérivés 4-sulfuriques de l'origine animale et du tissu d'extraction.

On a défini d'une façon certaine les différences entre les cartilages des trachées bovine et porcine. L'espèce bovine est plus riche en blocs de disaccharides 6-sulfatés que l'espèce porcine

### Exemple 4 : chromatogramme :

Le chromatogramme traduit les données suivantes :
Le pic 1 correspond à un bloc d'acide chondroïtine non sulfaté.
Le pic 2 correspond à un bloc d'acide chondroïtine sulfaté en 6.
Le pic 3 correspond à un bloc d'acide chondroïtine sulfaté en 4.
Le pic 4 correspond à un bloc d'acide chondroïtine non sulfaté et sulfaté en 4 en position terminale.
Le pic 6 correspond à un bloc d'acide chondroïtine sulfaté en 6 et en 4.
Le pic 8 correspond à un bloc d'acide chondroïtine sulfaté en 6 puis en 4 puis encore en 4 (6-4-4).
Le pic 9 correspond à un bloc d'acide chondroïtine sulfaté en 6 puis en 4 puis en 4 puis en 4 (6-4-4-4).
Le pic 10 correspond à un bloc d'acide chondroïtine sulfaté en 6 puis en 4 puis en 4 puis en 4 puis en 4 (6-4-4-4-4).
Le pic 11 correspond au bloc 6-4-4-4-4-4.
Le pic 12 correspond au bloc 6-4-4-4-4-4-4.
Le pic 13 correspond au bloc 6-4-4-4-4-4-4-4.
Le pic 14 correspond au bloc 6-4-4-4-4-4-4-4-4.
Le pic 15 correspond au bloc 6-4-4-4-4-4-4-4-4-4.

## Revendications

1. Blocs d'acides chondroïtine sulfuriques sulfatés en 4 et en 6, extraits des cartilages d'origine bovine et porcine, présentant un enchaînement et une nature linéaire déterminés par des méthodes physiques ou chimiques, et notamment après combinaison avec un réactif chromophore ou fluorescent.

2. Blocs d'acides chondroïtine sulfuriques sulfatés en 4 et en 6 selon la revendication 1, **caractérisés en ce que** leur identification a été réalisée par digestion enzymatique en utilisant la chondroïtin C-lyase, puis par chromatographie échangeuse d'anions et identification des fractions par fluorescence après combinaison avec un réactif chromophore.

3. Blocs d'acides chondroïtine sulfuriques sulfatés en 4 et en 6 selon les revendications 1 et 2, **caractérisés en ce qu'**ils comportent des blocs formés de deux, trois, quatre, cinq, six, sept, huit, neuf ou dix unités de disaccharides.

4. Blocs d'acides chondroïtine sulfuriques sulfatés en 4 et en 6 selon la revendication 1, **caractérisés en ce qu'**ils sont formés d'un enchaînement 6-4.

5. Blocs d'acides chondroïtine sulfuriques sulfatés en 4 et en 6 selon la revendication 1, **caractérisés en ce qu'**ils sont formés d'un enchaînement 6-4-4.

6. Blocs d'acides chondroïtine sulfuriques sulfatés en 4 et en 6 selon la revendication 1, **caractérisés en ce qu'**ils sont formés d'un enchaînement 6-4-4-4.

7. Procédé d'obtention des blocs d'acide chondroïtine sulfuriques sulfaté en 4 et en 6 selon l'une des revendications précédentes, qui comporte une phase de dépolymérisation des acides chondroïtine sulfuriques extraits de cartilage par action de la chondroïtine C-lyase, d'un fractionnement sur colonne de chomatographie d'échange d'anion et élution en milieu basique comportant un gradient de chlorure de sodium.

8. Compositions pharmaceutiques **caractérisées en ce qu'**elles renferment à titre de principe actif un bloc d'acide chondroïtine sulfurique selon la revendication 1 ou la revendication 2, en association ou en mélange avec un excipient ou un véhicule inerte, non toxique, pharmaceutiquement acceptable.

9. Utilisation comme médicament des blocs d'acides chondroïtine sulfuriques sulfatés en 4 et en 6 selon les revendications 1 à 7, notamment sous forme de compositions pharmaceutiques pour lutter contre les maladies articulaires liées au vieillissement.

10. Utilisation des blocs d'acides chondroïtine sulfuriques sulfatés en 4 et en 6 selon la revendication 9 par voie orale ou parentérale, sous forme de comprimés, de dragées, de gélules, de pilules, de cachets, de poudres aromatisées ou non, d'ampoules, de flacons monodoses ou de seringue auto-injectables.

11. Utilisation des blocs d'acides chondroïtine sulfuriques sulfatés en 4 et en 6 selon les revendications 9 et 10 **caractérisée en ce que** les doses usuelles pour la voie orale ou parentérale s'échelonnent de 0.1 à 2 g par prise unitaire.
